# FASCICULE DE BREVET EUROPEEN

(11) **EP 1 034 000 B1**
(45) Date de publication et mention de la délivrance du brevet: **31.01.2007**
(21) Numéro de dépôt: 98955673.3
(22) Date de dépôt: 17.11.1998
(51) Int. Cl.: A61K 39/21, C12N 15/48, C07K 16/10

(54) **OBTENTION DE VACCINS DESTINES A PREVENIR LES EFFETS PATHOGENES ASSOCIES A UNE INFECTION RETROVIRALE VIH**
HERSTELLUNG VON IMPSTOFFEN ZÜR VORBEUGUNG VON PATHOGENEN ZUSTÄNDEN EINER HIV RETROVIRALER INFEKTION
METHOD FOR OBTAINING VACCINES FOR PREVENTING THE PATHOGENIC EFFECTS RELATED TO A RETROVIRAL HIV INFECTION

(30) Priorité: 17.11.1997 FR 9714387
(43) Date de publication de la demande: 13.09.2000
(73) Titulaire: MYMETICS SA, 69230 Saint-Genis-Laval (FR)
(72) Inventeur: SERRES, Pierre-François, F-69230 Saint-Genis-Laval (FR); GEOURJON, Christophe, F-69007 Lyon (FR); DELEAGE, Gilbert, F-69008 Lyon (FR); COMBET, Christophe, F-42100 Saint-Etienne (FR)
(74) Mandataire: Le Coupanec, Pascale A.M.P.
(86) Numéro de dépôt international: PCT/FR1998/002447
(87) Numéro de publication internationale: WO 1999/025377

(56) Documents cités:
- WO-A-93/01304
- WO-A-94/02505
- WO-A-94/06471
- JOHNSON R P ET AL: "Identification of overlapping HLA class I-restricted cytotoxic T cell epitopes in a conserved region of the human immunodeficiency virus type 1 envelope glycoprotein: definition of minimum epitopes and analysis of the effects of sequence variation." JOURNAL OF EXPERIMENTAL MEDICINE, (1992 APR 1) 175 (4) 961-71, XP002073647

## Description

La présente invention concerne un procédé pour obtenir des vaccins destinés à prévenir les effets pathogènes associés, chez les humains ou chez les animaux vertébrés, à des infections rétrovirales.

Les effets pathogènes associés à une infection rétrovirale sont les effets néfastes, incluant d'éventuels effets oncogènes ou immunosuppresseurs, induits par l'introduction d'un rétrovirus dans l'organisme d'un hôte (mammifère, oiseau ou encore poisson), suivie de la pénétration et de la réplication dudit rétrovirus dans des cellules de l'hôte qui sont des cellules cibles du rétrovirus, c'est-à-dire des cellules dans lesquelles le virus est capable de pénétrer.

Les rétrovirus sont ainsi nommés parce qu'ils ont la capacité, grâce à l'enzyme appelée transcriptase inverse, d'effectuer une transcription d'ARN en ADN, alors que chez les êtres vivants l'information génétique va habituellement de l'ADN des chromosomes aux protéines, par l'intermédiaire de l'ARN messager.

Dans la famille des rétrovirus, on distingue trois sous-familles : les oncovirus, les lentivirus et les spumavirus.

Les oncovirus sont des rétrovirus ainsi nommés car ils peuvent être associés à des cancers et des infections malignes. On peut citer par exemple les virus leucémogènes (tels que le virus de la leucémie aviaire (ALV), le virus de la leucémie murine (MULV), encore nommé virus de Moloney, le virus de la leucémie féline (FELV), des virus de la leucémie humaine tels que HTLV1 et HTLV2, le virus de la leucémie simienne ou STLV, le virus de la leucémie bovine ou BLV), les oncovirus de type D des primates, les oncovirus de type B inducteurs de tumeurs mammaires, ou des oncovirus provoquant un cancer rapide (tel que le virus du sarcome de Rous ou RSV) ; voir par exemple STEHELIN et al., J. Mol.Biol. 101 : 349-365 (1976).

Les lentivirus sont ainsi nommés parce qu'ils sont responsables de pathologies à évolution lente impliquant très fréquemment des phénomènes immunosupresseurs, incluant des SIDAS.

Le Tableau 1 annexé indique à titre illustratif les pathologies associées à certains lentivirus, ainsi que les principales cellules cibles de ces lentivirus.

Les spumavirus manifestent assez peu de spécificité pour un type de cellule donné ou une espèce donnée, et ils sont parfois associés à des phénomènes immunosupresseurs; c'est le cas par exemple du virus spumeux du singe (ou SFV).

L'un des buts de la présente invention est l'élaboration de procédés et produits vaccinaux visant à prévenir efficacement les effets pathogènes, y compris les effets oncogènes ou immunosupresseurs, associés à l'infection d'un organisme-hôte par un rétrovirus.

L'immunosupression associée à l'infection a été constatée pour un très grand nombre de rétrovirus, et peut être considérée comme une constante pathogène de l'infection rétrovirale ; voir notamment BENDINELLI et al., Advances in Cancer Research 45 : 125-181 (1985). C'est le cas notamment des infections à lentivirus. C'est également le cas dans bon nombre d'infections à oncovirus; voir par exemple P. SONIGO dans l'ouvrage "SIDA et infection par VIH", MONTAGNIER et al. (Médecine Science Flammarion), pages 113-122 (1989).

De nombreux vaccins humains et animaux ont été essayés pour prévenir les effets pathogènes des infections à rétrovirus mais, en règle générale, ces vaccins sont peu efficaces ou inefficaces. En particulier, dans le domaine du SIDA humain ou animal, on constate que, 14 ans après la découverte du virus VIH (BARRE-SINOUSSI et al.. Science 220 : 860-871, 1983), on n'a pas encore pu trouver un vaccin parvenant à enrayer efficacement une infection post-vaccinale VIH ou VIS; voir par exemple LINHART et al., AIDS Research and Human Retroviruses 13 : 593-599 (1997); VOGT et al., Vaccine 13 : 202-208 (1995); et LETVIN et al., J. Virol. 69 : 4569-4571 (1995).

La majorité des préparations vaccinales utilisées comprennent des protéines de l'enveloppe rétrovirale sous différentes formes, par exemple, des virus inactivés, des protéines d'enveloppe comme les protéines gp 120 et gp 160 de VIH (voir notamment GORSE, G.J., vaccine 10 : 383-388, 1992), des cores de virus avec des protéines d'enveloppe, ou des protéines d'enveloppe associées à différents vecteurs (virus chimères, bactéries) ; voir Levy J.A., Trans.Med.Rev. 2: 265-271, 1988 et Microbiol.Rev.57: 183-289, 1993, en particulier page 247.

D'autres préparations utilisent des fragments de l'enveloppe rétrovirale ou des peptides immunodominants issus des glycoprotéines d'enveloppe, ces peptides étant présentés sous différentes formes (lipopeptides, peptides liés à une protéine support), de façon à les rendre immunogènes ; voir notamment Eriksson et al., Vaccine, 11 : 859-865 (1993).

Les stratégies vaccinales classiquement décrites, par exemple dans le domaine du SIDA humain, simien ou félin, préconisent de ne pas modifier les épitopes conservés et immunodominants des protéines d'enveloppe, ce qui peut sembler tout à fait logique. En effet, d'une part, ces épitopes conservés sont communs à différentes souches virales, ce qui est favorable à l'élaboration d'un vaccin qui doit induire une réponse immunitaire dirigée contre une majorité de souches. D'autre part, ces épitopes immunodominants sont bien reconnus par le système immunitaire, cellulaire ou humoral, lors du processus vaccinal et infectieux et, de surcroît, ils représentent fréquemment des sites de neutralisation ; voir par exemple HO et al., J.Virol. 61 : 2024-2028 (1987) ; JOHNSON et al., J.Exp.Med 175 : 961-971 (1992) ; SHAFFERMAN et al., P.N.A.S. U.S.A. 88 : 7126-7130 (1991) ; et HAMMOND et al., J.Immunol. 146 : 1470-1477 (1991).

Le Procédé de l'invention consiste, au contraire, à modifier des épitopes conservés et immunodominants de certaines protéines de l'enveloppe virale, afin d'aboutir à un vaccin efficace.

En effet, les auteurs de la présente invention ont découvert que des zones conservées et immunodominantes de l'enveloppe rétrovirale peuvent être à l'origine de phénomènes auto-immuns nuisibles. A titre d'exemple, dans le cas du SIDA humain, ils ont observé que certaines zones conservées et immunodominantes de l'enveloppe du VIH présentent des analogies de structure tridimensionnelle et/ou des réactions croisées avec certaines zones d'au moins une protéine du système immunitaire humain, de sorte que l'administration comme vaccin d'une protéine virale contenant lesdites zones intactes induit une réponse immunitaire qui est à l'origine de réactions auto-immunes néfastes conduisant à l'échec vaccinal.

A l'origine de la présente invention, on trouve, d'une part, l'observation mentionnée précédemment que des zones conservées et immunodominantes de certains rétrovirus, présentes habituellement dans des préparations vaccinales, sont précisément, dans bon nombre de cas, des zones qui provoquent des réactions auto-immunes néfastes parce qu'elles présentent des analogies de structure tridimensionnelle et/ou des réactions croisées avec certaines protéines de l'hôte du virus. A l'origine de la présente invention, on trouve aussi, d'autre part, l'observation que lesdites protéines de l'hôte utilisent la même cellule cible, ou les mêmes cellules cibles, que lesdits rétrovirus. L'ensemble de ces observations effectuées par les auteurs de l'invention les ont conduit à penser que les protéines d'enveloppes rétrovirales et les protéines de l'hôte qui présentent des analogies de structure tridimensionnelle et/ou des réactions croisées se fixent dans de nombreux cas sur les mêmes cellules cibles et possèdent, sur ces cellules cibles, des récepteurs membranaires communs.

On dit qu'une protéine présente une réaction croisée avec une autre protéine lorsqu'il est possible d'obtenir, par immunisation *in vivo* ou *in vitro* à l'aide de l'une desdites protéines, une réponse immunitaire dirigée aussi contre l'autre protéine, par exemple lorsque cette immunisation induit une réponse humorale (dite de type B) et permet d'obtenir et de sélectionner au moins un anticorps monoclonal qui est capable de reconnaître l'autre protéine, ou lorsqu'une même réponse immunitaire cellulaire (c'est-à-dire de type T) induite *in vitro* par l'une des protéines reconnaît les deux protéines, selon les tests connus de mise en évidence d'une réponse immunitaire de type T, tels que par exemple les tests de cytotoxicité *in vitro.* On sait que le terme "immunisation" désigne le processus d'induction d'une réponse immunitaire consécutive à la stimulation, par mise en contact *in vivo* ou *in vitro,* de cellules immunocompétentes d'un hôte avec un antigène, et que l'un des buts de l'administration d'un agent vaccinal est justement d'obtenir une telle immunisation.

L'invention a donc pour objet un procédé d'obtention d'un vaccin contre les effets pathogènes associés à l'infection d'un hôte, animal ou humain, par un rétrovirus VIH capable de pénétrer dans une cellule cible dudit hôte, ladite cellule cible possédant un récepteur membranaire pour la protéine de l'hôte interleukine-2 (IL-2), procédé dans lequel on prépare un agent vaccinal à base d'un polypeptide comprenant au moins une partie de la protéine d'envelopper gp41 d'une souche pathogène dudit rétrovirus, et dans lequel ledit polypeptide est préparé sous une forme modifiée, étant entendu que :
- ladite partie de la protéine d'enveloppe gp41 est choisie parmi celles qui comprennent au moins un fragment d'une zone immunodominante de ladite protéine d'envelopper ledit fragment contenant au moins un acide aminé qui est un acide aminé conservé de ladite zone immunodominante et qui est présent dans ladite souche pathogène,
- ledit polypeptide, à l'état non modifié, induit une réponse immunitaire dirigée à la fois contre ladite zone immunodominante et contre la protéine de l'hôte IL-2,
- et ledit polypeptide modifié est choisi par ceux qui induisent une réponse immunitaire dirigée contre ladite zone immunodominante de la protéine d'enveloppe gp41 et non contre la protéine de l'hôte IL-2.

Dans la définition du procédé de l'invention qui vient d'être donnée, l'agent vaccinal est dit "à base" d'un polypeptide' modifié. Cela signifie que l'agent vaccinal comprend un tel polypeptide modifié, mais cela ne signifie pas que l'agent vaccinal est nécessairement de nature exclusivement polypeptidique. En fait, dans cet agent vaccinal, ledit polypeptide peut, éventuellement, être lié (notamment de façon covalente) ou associé, de façon connue en soi, à toute molécule biocompatible qui peut être choisie, par exemple, parmi les polymères, les lipides, les peptides (y compris des lipopeptides, des glycopeptides, des protéines), les acides nucléiques, les oligosaccharides, etc. Ladite molécule biocompatible peut notamment servir de support à l'agent immunogène polypeptidique. Elle peut aussi servir à modifier la conformation du polypeptide et, dans ce dernier cas, ladite molécule doit être considérée comme un substituant modifiant le résidu d'acide aminé auquel il est attaché, ledit substituant modifiant ainsi, en définitive, l'antigénicité du polypeptide dont ce résidu d'acide aminé fait partie.

Le procédé de l'invention peut comprendre, au moins dans une phase préliminaire de recherche, une étape consistant à sélectionner des polypeptides (non modifiés) comprenant au moins une partie, telle que définie ci-dessus, de la protéine d'enveloppe virale gp41 d'une souche pathogène du rétrovirus VIH. Cette partie de protéine, qui comprend au moins un fragment immunogène d'une zone immunodominante, est telle que le polypeptide (non modifié) est capable d'induire une réponse immunitaire dirigée à la fois contre la protéine virale (plus précisément contre le fragment de la zone immunodominante contenu dans ladite partie) et contre la protéine de l'hôte IL-2, et c'est l'existence d'une telle réponse immunitaire, dirigée contre la protéine d'enveloppe virale gp41 et contre la protéine de l'hôte IL-2, qui définit, dans la présente demande, le caractère pathogène d'une souche virale. On peut ainsi sélectionner les polypeptides (non modifiés) comprenant un tel fragment.

un fragment polypeptidique est dit immunogène si l'immunisation d'un hôte, *in vivo* ou *in vitro,* avec ledit fragment, éventuellement lié à un support approprié (tel qu'une protéine, un lipide ou un polypeptide), permet d'obtenir une réponse immunitaire, de type B et/ou de type T, dirigée contre ledit fragment polypeptidique.

Dans la présente demande, lorsqu'on parle d'une réponse immunitaire, sans autres précisions, il s'agit d'une réponse immunitaire d'un vertébré, consécutive à une immunisation *in vitro* ou *in vivo.*

Le procédé de l'invention peut aussi comprendre au moins une étape consistant à modifier, de la façon qui sera indiquée ci-après, un polypeptide ainsi sélectionné, et à choisir, parmi les polypeptides ainsi modifiés, au moins un polypeptide modifié qui induit une réponse immunitaire dirigée contre la protéine d'enveloppe virale gp41 non contre la protéine de l'hôte IL-2.

Ainsi, alors que l'art antérieur enseignait, comme noté ci-dessus, de ne pas modifier les épitopes conservés et immunodominants des protéines d'enveloppes rétrovirales, le procédé de l'invention a pour but, au contraire, de modifier l'antigénicité de tels épitopes de façon à obtenir une réponse immunitaire différenciée vis-à-vis de la protéine d'enveloppe virale et d'une protéine de l'hôte.

On sait que pour modifier l'antigénicité d'un fragment immunogène d'un polypeptide, il est possible de modifier ledit polypeptide à l'aide d'une mutation portant sur au moins un acide aminé. On définira plus loin ce qu'il faut entendre ici par "mutation". L'acide aminé muté peut être situé dans le fragment immunogène, ou même dans une zone du polypeptide extérieure audit fragment. On sait en effet que la modification d'un acide aminé situé à l'extérieur d'un fragment peut affecter la structure spatiale dudit fragment, et donc son antigénicité ; en particulier, il a été montré que la conformation d'un résidu d'acide aminé, dans un peptide, peut être influencée par la nature des résidus d'acides aminés à des positions allant de +8 à -8 par rapport à ce résidu d'acide aminé ; voir par exemple GARNIER et al., J.Mol.Biol. 120 : 97-120 (1978). Au-delà, la nature des résidus d'acides aminés a encore une influence, mais cette influence n'est ni systématique ni quantifiable à partir de la seule connaissance de la séquence peptidique considérée.

Un acide aminé muté peut donc être situé, dans le polypeptide modifié, à l'intérieur ou à l'extérieur du fragment immunogène. Lorsqu'il est à l'extérieur du fragment immunogène, il n'est généralement pas séparé de l'extrémité la plus proche dudit fragment immunogène, dans la chaîne polypeptidique, par plus de huit (et notamment par plus de sept) résidus d'acides aminés. En particulier, un acide aminé, muté conformément à la présente invention, et situé à l'extérieur du fragment immunogène, n'est généralement pas séparé par plus de huit résidus d'acides aminés, et en particulier par plus de sept résidus d'acides aminés, de l'acide aminé conservé le plus proche appartenant à la zone inmunodominante dont au moins un fragment est contenu dans le polypeptide non modifié.

Le polypeptide modifié conformément à la présente invention peut être par exemple la protéine d'enveloppé JP61 entière d'une souche virale VIH pathogène, modifiée par au moins une mutation comme indiqué ci-dessus. Le polypeptide modifié peut être aussi une partie de la protéine d'enveloppe gp41 d'une souche virale VIH pathogène, modifiée par au moins une mutation comme indiqué précédemment, ladite partie comprenant au moins un fragment immunogène tel que défini précédemment. Le polypeptide modifié peut être également une protéine chimère comprenant au moins une partie de la protéine d'enveloppe gp41 ladite partie de la protéine d'enveloppe étant définie comme précédemment et comportant au moins une mutation.

La définition donnée ci-dessus du procédé de l'invention implique que le polypeptide utilisé comprenne au moins une partie d'une zone immunodominante et conservée d'une protéine d'enveloppe virale gp41. Dans la description de la présente demande, on appelle "zone conservée" une zone, éventuellement réduite à un seul résidu d'acide aminé, de la protéine virale, où l'on retrouve, pour une majorité de souches d'un virus donné (par exemple dans au moins 6 souches sur 10 environ), un ou plusieurs acides aminés identiques ou fonctionnellement analogues situés à la même position dans des alignements de séquences peptidiques de ladite protéine des diverses souches. Un tel acide aminé identique ou fonctionnellement analogue est appelé acide aminé conservé. La notion de conservation d'acides aminés fonctionnellement analogues est connue, et il existe de nombreuses matrices de substitutions permettant de quantifier cette notion (Dayhoff, M.O. et al., in Atlas of Protein Séquence and Structure, 1978, vol 5, Suppl 3, Chapitres 22 et 23).

Les zones conservées peuvent être facilement déterminées, après séquençage de protéines de diverses souches du virus étudié, par les méthodes d'alignements multiples des séquences obtenues. On peut utiliser pour cela par exemple le programme Clustal-w (Thompson J.D. et al., Nucleic Acids Research 22 : 4673 - 4680, 1994). Par ailleurs, les séquences de protéines de diverses souches virales sont souvent accessibles sur des bases de données. Par exemple, le serveur Web de la base de données VIH de Los Alamos présente les séquences VIH1 et VIH2 remises à jour régulièrement. L'adresse de ce serveur sur le réseau Internet est:
http://hiv-web.lan1.gov/HTML/sequences.html

Les tableaux 2a, 2b, et 2c annexés sont des exemples d'alignement de séquence des régions appartenant aux glycoprotéines d'enveloppes homologues de la région 545-682 de la glycoprotéine transmembranaire de VIH1 (entrée SWISSPROT ENV_HV1 BR), respectivement pour VIH1 et VIH2. La dernière ligne des tableaux résume, à l'aide de symboles, le degré d'homologie, et donc le degré de conservation, observé. Le symbole "*" indique une position de l'alignement où le même résidu est présent dans toutes les séquences, le symbole ":" indique une position dans l'alignement où les acides aminés présents dans les différentes séquences sont très similaires, le symbole "." indique une position dans l'alignement où les acides aminés présents dans les différentes séquences sont similaires, et l'absence de symbole indique une position dans l'alignement où les acides aminés présents dans les différentes séquences sont peu similaires. Cette symbolique est utilisée par le programme d'alignement Clustal w (version 1.7).

Dans la description de la présente demande, on appelle zone immunodominante d'une protéine une séquence peptidique qui induit dans une grande majorité des cas (par exemple dans au moins 7 cas sur 10 environ) une réponse humorale et/ou cellulaire du système immunitaire dirigée contre ladite zone après immunisation avec une protéine contenant ladite séquence ou avec un peptide constitué essentiellement par ladite séquence.

La définition du procédé de l'invention fait référence aux cellules cibles d'un virus qui sont les cellules à l'intérieur desquelles le virus est capable de pénétrer. Les cellules cibles des rétrovirus sont généralement connues. Les virus ont la propriété de se fixer sur les cellules qu'ils sont susceptibles d'infecter. On peut donc éventuellement rechercher par des expériences de routine *in vitro* les cellules cibles d'un virus étudié.

La définition du procédé de l'invention fait aussi référence aux cellules de l'hôte ayant un récepteur membranaire pour une protéine de l'hôte IL-2. Les cellules de l'hôte ayant un récepteur pour une protéine de l'hôte IL-2 sont souvent connues et, dans le cas contraire, il est possible, par des expériences de routine, de déterminer si une protéine donnée se fixe sur un certain type de cellules. On peut par exemple utiliser une protéine radiomarquée et déterminer si elle se fixe sur ledit type de cellule. On peut également rechercher si la protéine se fixe sur un récepteur membranaire donné en utilisant une lignée cellulaire transfectée par un gène exprimant ledit récepteur membranaire.

Dans la présente demande, on considère qu'une réponse immunitaire, par exemple une réponse anticorps, obtenue par immunisation à l'aide du polypeptide modifié préparé conformément au procédé de l'invention, est dirigée contre la protéine d'enveloppe virale gp41 et non contre la protéine de l'hôte IL-2, lorsque les anticorps obtenus ont des affinités pour la protéine de l'hôte et pour la protéine d'enveloppe du rétrovirus qui présentent une différence importante, se traduisant notamment par des différences de réactivité considérées comme très significatives dans des tests ELISA, telles que par exemple des densités optiques dans un rapport d'environ 4 (ou davantage), ce qui signifie que la densité optique observée après fixation desdits anticorps sur la protéine virale gp41 est au moins quatre fois plus élevée que celle observée pour la fixation desdits anticorps sur la protéine de l'hôte IL-2. De façon analogue, une réponse immunitaire de type cellulaire est considérée comme dirigée contre la protéine d'enveloppe gp41 mais non contre la protéine de l'hôtel IL-2 lorsque l'immunisation *in vitro* de cellules immunocompétentes de l'hôte avec le candidat vaccin induit la formation de cellules activées dont la réaction vis-à-vis de cellules (incluant des lignées cellulaires transfectées) exprimant la protéine d'enveloppe rétrovirale est significativement plus élevée que la réaction vis-à-vis de cellules exprimant la protéine de l'hôte, par exemple lorsque, dans la mesure optique finale, ou dans le comptage final de radioactivité (notamment radioactivité de ⁵¹Cr relargué par des cellules cibles) du test mis en oeuvre, ou encore dans l'appréciation par tous moyens connus d'une lyse cellulaire causée par des cellules cytotoxiques induites, les échelles de réponse sont dans un rapport d'environ 4 (ou davantage). Les critères qui viennent d'être indiqués permettent au moins de faire un premier choix parmi les peptides modifiés étudiés, mais en définitive c'est l'absence ou la diminution de l'effet pathogène dû à la suppression ou à l'affaiblissement (mis en évidence par tout moyen approprié) de la réponse immunitaire vis-à-vis de la protéine de l'hôte, qui constituera le critère de sélection des peptides modifiés susceptibles de constituer des agents vaccinaux satisfaisants.

Les zones immunodominantes et conservées dont on souhaite modifier l'antigénicité, conformément à l'invention, peuvent être choisies parmi celles qui donnent *in vitro* une réaction croisée, de type B et/ou de type T, avec la protéine de l'hôte IL-2.

On peut aussi choisir une telle zone immunodominante et conservée parmi celles pour lesquelles on a déterminé préalablement une analogie de structure tridimensionnelle avec une zone de la protéine de l'hôte IL-2, ladite analogie de structure étant susceptible d'être associée à une réaction croisée *in vitro* et/ou *in vivo.* L'analogie de structure tridimensionnelle entre certaines zones de deux protéines fait référence à des dispositions équivalentes, dans l'espace, de résidus d'acides aminés qui sont similaires en raison notamment de leur chaîne latérale et/ou de leurs groupements chimiques fonctionnels analogues. Les structures tridimensionnelles des protéines peuvent être obtenues à l'aide de spectres de résonance magnétique nucléaire (RMN) et/ou de spectres de diffraction des rayons X. Par exemple, la structure de la protéine gp41 de VIS a été obtenue à l'aide du spectre RMN (Caffrey M. et al., J. Mol.Biol. 271, 819-826, 1997). En outre, il est possible, dans certains cas, d'obtenir un bon modèle à l'aide de techniques de modélisation moléculaire, à partir des coordonnées atomiques d'une protéine de structure connue. On peut utiliser pour cela, notamment, le logiciel de modélisation moléculaire X-plor (référence : "A system for X-ray crystallography and NMR, Version 3.1", Axel T. Brunger, Yale Unversity Press, 1992).

Pour rechercher une analogie de structure tridimensionnelle, on peut faire appel par exemple aux méthodes connues de visualisation et de superposition sur écran graphique de la structure tridimensionnelle de molécules biologiques. Il existe des logiciels qui permettent la visualisation des structures tridimensionnelles des molécules avec différents modes de représentation, le calcul de paramètres géométriques (tels que distances, angles, etc) et la superposition objective et quantitative de plusieurs structures moléculaires (notamment logiciels RASMOL : Sayle, R.A. et Milner-White E. J., J. Mol. Biol., 247, 536-540, 1995 et ANTHEPROT : Geourjon C. et Deléage G., J. Mol. Graph. 13, 209-212, 1995) ainsi que l'estimation de l'accessibilité aux solvants (logiciels X-plor, déjà mentionné, et CCP4 : Collaborative Computational Project Number 4, *Acta Cryst.,* D50, 760-763, 1994.

Cependant, afin d'avoir une estimation plus fine de ces analogies structurales, il est utile de considérer, au niveau de chaque acide aminé, les groupements fonctionnels positionnés de manière analogue dans les deux protéines que l'on compare. Pour cela, les co-inventeurs de la présente invention utilisent des méthodes permettant de calculer des surfaces moléculaires dans le but de comparer des propriétés fonctionnelles entre deux structures tridimensionnelles, pour tenir compte non pas des acides aminés dans leur globalité, mais aussi, plus spécialement, des groupements chimiques fonctionnels de chacun d'entre eux (par exemple : fonctions amide, carboxylique, hydroxyle, sulfhydryle, amine, etc.). On peut ainsi prendre en considération, dans les structures comparées, des acides, aminés fonctionnellement analogues, et non pas seulement des acides aminés identiques

On considère donc qu'une zone d'une protéine rétrovirale gp41 présente une analogie de structure tridimensionnelle avec une zone donnée d'une protéine de l'hôte IL-2, lorsque les techniques qui viennent d'être mentionnées permettent de mettre en évidence, dans les deux zones comparées, une organisation spatiale similaire de certains acides aminés identiques ou fonctionnellement analogues.

Il est à noter que les acides aminés fonctionnellement analogues et regroupés de façon similaire dans l'espace peuvent être relativement éloignés les uns des autres dans une même chaîne peptidique. Mais l'analogie de structure tridimensionnelle, entre deux protéines que l'on compare, peut aussi concerner la disposition dans l'espace, de façon similaire, d'acides aminés identiques ou fonctionnellement analogues dans le cas où, l'une des protéines étant oligomérisée, les résidus d'acides aminés impliqués sont situés sur des chaînes différentes de l'oligomère, tandis que les résidus d'acides aminés de l'autre protéine qui sont impliqués dans cette analogie peuvent être situés sur une même chaîne peptidique de cette autre protéine.

Il est particulièrement avisé de rechercher des analogies de structure tridimensionnelle et/ou des réactions croisées avec des zones de la protéine de l'hôte IL-2 impliquées dans la fixation de ladite protéine sur son récepteur.

Pour préparer le polypeptide modifié qui constitue l'agent vaccinal obtenu selon l'intention, on peut utiliser les méthodes connues de synthèse peptidique ou les méthodes du génie génétique. On peut isoler ou préparer une séquence polynucléotidique codant pour au moins une partie de la protéine d'enveloppé gp41 du virus et, si désiré, on peut introduire à ce stade, dans la séquence nucléotidique, les mutations permettant d'obtenir un produit de traduction muté qui constitue le polypeptide modifié. On peut également synthétiser directement une séquence polynucléotidique modifiée comportant une ou plusieurs mutations et codant pour le polypeptide modifié. Les séquences polynucléotidiques mutées ainsi obtenues sont introduites de façon connue dans un vecteur approprié permettant d'exprimer ledit polypeptide, éventuellement sous forme modifiée. Un tel vecteur est par exemple E. coli, un baculovirus, ou une cellule de mammifère. On peut aussi effectuer la mutation sur un polypeptide non modifié obtenu selon l'une des méthodes précédentes.

Dans la présente demande, on appelle "mutation" toute modification d'une région (éventuellement réduite à un seul résidu d'acide aminé) d'un polypeptide, par des moyens physiques, des moyens chimiques (modification covalente ou non covalente) et/ou biologiques (mutations par substitution, délétion et/ou insertion d'un ou plusieurs acides aminés), conduisant à la modification des potentialités fonctionnelles du ou des acides aminés constitutifs de ladite région, dite "région mutée". A titre d'exemple, on peut effectuer des modifications conduisant à l'abolition, l'acquisition et/ou la modulation des propriétés de ponts disulfure, de liaisons hydrogène, d'interactions électrostatiques et/ou d'interactions hydrophobes, la modification de la capacité d'une protéine à former un hétéro-complexe, ou encore, dans le cas d'une protéine oligomère, la modification de l'état d'oligomérisation ou de la stabilité.de l'oligomère.

La modification d'un acide aminé a d'une chaîne polypeptidique (y compris la modification d'un acide aminé terminal du polypeptide considéré) peut influencer la conformation des acides aminés voisins dans la chaîne, y compris, comme on l'a rappelé ci-dessus, la conformation d'un acide aminé b séparé de a par un nombre de résidus d'acides aminés pouvant aller jusqu'à sept ou huit, et lorsque l'acide aminé b fait partie d'un épitope, toute modification de l'acide aminé a (notamment toute addition d'un substituant ou toute modification d'un substituant) est susceptible de modifier l'antigénicité de l'épitope considéré.

Dans la phase de recherche de polypeptides modifiés conformément à l'invention, le choix des acides aminés à muter et/ou le choix des méthodes de mutation peut être fait de façon arbitraire ou de façon raisonnée. On peut utiliser notamment l'une au moins des méthodes de modification suivante :
1) le remplacement d'un ou plusieurs acides aminés ayant une chaîne latérale hydrophobe (Exemples: Ala, Leu, Val, Ile, Phe, Trp, Met, Tyr, Cys) par un ou plusieurs acides aminés ayant une chaîne latérale hydrophile (Exemples : Arg, Asp, Asn, Glu, Gln, Ser, Thr, His, Lys) ou indifférente (Exemples: Gly, Pro) et *vice versa;*
2) le remplacement d'un ou plusieurs acides aminés ayant une chaîne latérale positivement chargée (Exemples : Arg, Lys, His) par un ou plusieurs acides aminés ayant une chaîne latérale négativement chargée (Exemples : Asp, Glu) ou neutre et *vice versa ;*
3) l'acquisition, la suppression et/ou la modification d'un ou plusieurs ponts disulfure;
4) la réalisation de mimotopes, en particulier obtenus par *retro inverso ;*
5) les substitutions, suppressions, additions et/ou autres modifications d'au moins un acide aminé potentiellement donneur ou accepteur de liaisons hydrogène ;
6) les substitutions, suppressions, additions et/ou autres modifications d'au moins un acide aminé potentiellement donneur ou accepteur de liaisons ioniques;
7) le changement de l'encombrement stérique par substitutions, suppressions, additions et/ou autres modifications d'un ou plusieurs acides aminés ;
8) l'utilisation d'acides aminés non naturellement présents dans les protéines ;
9) la modification de la glycosylation (création, suppression ou modification de sites de glycosylation ou de leurs sucres associés).

Bien entendu, les polypeptides modifiés ainsi obtenus sont testés, comme indiqué ci-dessus, afin de sélectionner les polypeptides modifiés qui induisent une réponse immunitaire dirigée contre la protéine d'enveloppe gp41 et non contre la protéine de l'hôte IL-20.

Le procédé de l'invention peut être appliqué à l'obtention de vaccins, notamment contre les virus suivants : VIH.

L'invention a également pour objet l'utilisation d'un polypeptide modifié, tel que défini précédemment, dans la préparation d'une composition vaccinale destinée à prévenir les effets pathogènes associés à l'infection d'un hôte par un rétrovirus VIH.

L'invention concerne également une composition vaccinale pouvant être obtenue par le procédé de l'invention, et contenant comme ingrédient actif un polypeptide modifié tel que décrit, ci-dessus. Une telle composition peut être utilisée dans une méthode de vaccination destinée à prévenir les effets pathogènes des infections à rétrovirus VIH, cette méthode consistant essentiellement à administrer à un animal vertébré, y compris un humain, un polypeptide modifié tel que défini ci-dessus en quantité suffisante pour obtenir un effet de vaccination. La formulation des compositions vaccinales, et leur méthode d'administration, sont connues en soi et ne seront pas décrites davantage ici.

L'invention a aussi pour objet un polynucléotide rétroviral modifié codant pour un polypeptide modifié tel que défini précédemment. Le polynucléotide modifié peut être obtenu comme indiqué précédemment. L'invention s'étend à un vecteur d'expression dans lequel ledit polynucléotide modifié a été inséré, ledit vecteur d'expression étant ainsi capable d'exprimer le polypeptide modifié.

Le polypeptide modifié obtenu selon l'invention peut également servir d'agent immunogène en vue d'induire par immunisation la formation d'anticorps utilisables notamment dans le traitement des infections rétrovirales, et l'invention concerne donc également des anticorps obtenus en réponse à l'immunisation d'animaux (y compris des humains), *in vivo* ou *in vitro*, à l'aide de l'agent vaccinal contenant un polypeptide modifié décrit ci-dessus. Les anticorps de l'invention sont notamment des anticorps polyclonaux purifiés ou des anticorps monoclonaux présentant la caractéristique de reconnaître la protéine d'enveloppe rétrovirale sans reconnaître la protéine de l'hôte. La purification des anticorps polyclonaux, et la sélection des anticorps monoclonaux, peuvent être effectués à l'aide de la protéine virale et de la protéine de l'hôte, de façon à ne retenir que les anticorps reconnaissant la protéine virale et non la protéine de l'hôte. Les anticorps de l'invention peuvent être utilisés notamment dans le traitement précoce des infections provoquées chez ledit hôte par le rétrovirus contre lequel ils sont dirigés. La posologie est la posologie usuelle des anticorps. Les compositions pharmaceutiques contenant de tels anticorps constituent également l'un des objets de l'invention.

Les exemples suivants illustrent l'invention.

### EXEMPLE 1

On expose ci-après comment les auteurs de la présente invention ont recherché des analogies structurales et antigéniques entre une protéine d'enveloppe d'un virus, à savoir la protéine gp41 du VIH 1, et une cytokine, à savoir l'interleukine-2 (en abrégé : IL-2) humaine.

On remarquera que les séquences peptidiques de gp41 et de l'IL-2 humaine ne sont pas homologues. En effet, les deux protéines ne présentent globalement entre elles que 16,5% d'identité de séquence, ce seuil d'homologie n'étant pas significatif, comme on peut le constater aisément à l'aide de simulations in silico réalisées sur des séquences de même composition mais dont l'ordre des acides aminés a été aléatoirement modifié.

Des travaux anciens (Bost et al., Immunology 65 : 611-615, 1988) avaient signalé une homologie de séquence entre la protéine gp41 et l'IL-2 (séquence LERILL). Il faut noter que cette séquence LERILL de gp41 ne constitue pas une zone immnunodominante de cette protéine ; voir LEVY J.A, Microbiol. Rev. 57 : 183-289 (1993) en particulier page 232. La séquence LERILL est d'ailleurs située à l'intérieur de la particule virale ; elle correspond à la partie C-terminale de gp41.

Ayant observé que l'IL-2 et les rétrovirus associés aux SIDAS semblent avoir des cellules cibles communes, les auteurs de la présente invention ont fait l'hypothèse que le récepteur de l'interleukine-2 humaine pourrait être commun à l'IL-2 et à la protéine gp41 du VIH et ils ont donc recherché d'éventuelles analogies structurales tridimensionnelles entre ces deux dernières.

Dans la présente demande, les numérotations des résidus d'acides aminés de la séquence peptidique de l'interleukine-2 et de gp41 sont celles utilisées dans la banque SWISSPROT (version 34).

Les séquences peptidiques de l'IL-2 et de la protéine gp41 sont connues. Dans la présente demande, on fait référence aux séquences publiées suivantes :
pour IL-2: entrée SWISSPROT (version 34) ayant pour code IL2_HUMAN;
- pour gp41 : entrée SWISSPROT (version 34) ayant pour code ENV_HV1BR.

Les structures publiées qui ont été utilisées sont les suivantes :
- pour IL-2 : entrée PDB (Brookhaven Databank) 1IRL ;
- pour gp41 : entrées PDB (Brookhaven Databank)
   - 1AIK,
   - 1ENV.

Par ailleurs, la structure tridimensionnelle de l'IL-2, déterminée par RMN, est connue (Mott, P.C. et al., J. Mol. Biol., 248 : 979,1995), de même que la structure de certains domaines de la protéine gp41, qui a été obtenue à l'aide du spectre de diffraction des rayons X (Chan, D.C. et al., Cell, 89, 263-273, 1997 ; Weissenhorn, W et al., Nature, 387, 426-430, 1997). D'autre part, un modèle tridimensionnel d'une partie du domaine externe, dans la région 545-671, de la protéine gp41 (forme trimère) a été obtenu, par modélisation moléculaire, par les co-inventeurs de la présente invention. Ce modèle moléculaire a été obtenu en utilisant le logiciel X-plor par une stratégie proche de celle de la modélisation moléculaire sous contraintes RMN. Les contraintes nécessaires pour la modélisation moléculaire de la forme trimère ont été déduites de la structure tridimensionnelle du mutant pII du domaine "leucine zipper" de la protéine GCN4 (code PDB : 1GCM), cristallisé sous la forme d'un trimère de type "coiled coil".

En examinant les structures obtenues, des analogies tridimensionnelles ont été trouvées entre certaines zones de la protéine gp41 et certaines zones de l'interleukine-2 participant à la fixation sur son récepteur. Le mode de fixation de l'IL-2 sur son récepteur, ainsi que les zones de l'IL-2 impliquées dans cette fixation, sont en effet connus ; voir BAZAN J.F., P.N.A.S. USA 87 : 6934-6938 (1990); Bamborough P. et al., Structure 2 : 839-851 (1994); Gnarra J., R. et al., P.N.A.S. USA 87: 3440-3444 (1990) ; Takeshita T. et al., Science 257 : 379-382 (1992) ; et CAVAILLON J.M., Les Cytokines (Masson, Paris, 1996), pages 119-125.

Ces résultats ont été confirmés par des' études de comparaisons globales des structures de gp41 et de l'IL-2, et aussi par des comparaisons locales faites en s'intéressant plus spécialement aux groupements fonctionnels analogues de chacune des structures, comme déjà indiqué dans la description ci-dessus.

On a observé notamment que les régions 53-61 et 88-93 de l'IL-2, organisées en hélice alpha, se superposent de façon satisfaisante avec deux des trois hélices du trimère central de gp41. Cela implique que dans les deux protéines, des groupements portés par des hélices différentes peuvent avoir des propriétés d'accessibilité et d'organisation relative comparables.

Il a également été trouvé des analogies structurales tridimensionnelles locales entre une région immunodominante fortement conservée de la glycoprotéine gp41 du VIH (plus précisément dans la région 545-682) et l'interleukine-2 humaine.

La séquence peptidique de la région 545-682 de la protéine gp41 du VIH 1 (code SWISSPROT : ENV_HV1BR) est reproduite dans le Tableau 3 annexé.

Dans le Tableau 3bis annexé, on a représenté les séquences peptidiques de quatre zones de cette région de gp41 (555-577, 572-601, 590-620 et 628-663), dans lesquelles on a relevé des analogies structurales et/ou des réactions croisées avec l'IL-2.

Les zones de l'IL-2 concernées par les analogies de structure qui viennent d'être mentionnées sont les zones 27-47, 45-69, 99-121 et 131-153 de l'IL-2. Les séquences peptidiques de ces zones sont représentées dans le Tableau 4 annexé.

Il est important de noter que la zone 27-47 de l'IL-2 est impliquée dans la fixation de l'IL-2 sur la chaîne bêta de son récepteur. En effet, les acides aminés dans la zone 27-47 appartiennent à l'hélice A qui participe à la fixation sur le récepteur de l'IL-2 (RIL-2), plus précisément sur bêta RIL-2.

Les acides aminés dans la zone 45-69 appartiennent à une région de l'IL-2 qui participe à la fixation sur alpha RIL-2.

Les acides aminés dans la région 99-121 appartiennent à l'hélice E participant à la fixation sur bêta RIL-2.

Les acides aminés dans la zone 131-153 de l'IL-2 appartiennent à l'hélice F participant à la fixation sur gamma RIL-2.

A titre illustratif, on précise dans le Tableau 4bis annexé les analogies des structures qui ont été trouvées entre la zone 572-601 de gp41 et la zone 27-47 de l'IL-2 humaine. Les acides aminés externes impliqués dans cette analogie de structure tridimensionnelle sont soulignés dans le Tableau 4bis.

Il faut noter cependant qu'une même zone de gp41 peut présenter des analogies de structure tridimensionnelle avec plusieurs zones distinctes de l'IL-2.

En outre, les auteurs de l'invention ont remarqué que dans la région 600-612 de gp41, les trois lysines (K) en position 606 sur les trois chaînes de trimère de gp41 sont susceptibles de former un épitope conformationnel, ces lysines de gp41 pouvant correspondre, dans l'espace, aux lysines 52, 96 et 55 de l'IL-2.

Ils ont également observé des réactivités immunologiques croisées entre les protéines gp41 et IL-2. En particulier, en utilisant les techniques ELISA et PEPSCAN, avec des anticorps provenant de sérums VIH purifiés par immunopurification sur une colonne contenant de l'IL-2 humaine immobilisée, ils ont constaté que certains de ces anticorps reconnaissent des zones de l'IL-2 impliquées dans la fixation de l'IL-2 sur les chaînes alpha, bêta et gamma de son récepteur, et notamment des zones appartenant à l'hélice A (KTQLQLEHLLLTLQ), l'hélice E (RFMLISNINVIVLELK), l'hélice F (TIVEFLNRWITFCQSIISTLT), la boucle AB et le début de l'hélice B (NNYKNPKLTRMLTFKFYMFKK).

En utilisant les techniques de dépôt Ponctuel sur filtre (ou "dot") et les techniques d'immunotransfert du type western blot, il a également été montré que des anticorps polyclonaux provenant de sérums de malades VIH+ et immunopurifiés sur de l'IL-2 humaine, reconnaissent des oligomères de la protéine gp41.

Les études effectuées ont également montré que des anticorps monoclonaux anti-gp41 murins et humains dirigés contre des zones conservées immunodominantes de la protéine gp41 des VIH reconnaissent des zones de l'IL-2 qui participent à la fixation de cette dernière sur les chaînes alpha, bêta et gamma du récepteur de l'IL-2.

On peut donc obtenir des vaccins contre le virus VIH conformément à l'invention, notamment en préparant des polypeptides contenant au moins l'une des zones de gp41 décrites dans le Tableau 3bis, lesdits polypeptides étant sous forme modifiée, c'est-à-dire contenant au moins une mutation, comme indiqué précédemment. Il faut bien comprendre que ces découpages de la région 545-652 en zones peuvent présenter un certain caractère arbitraire, et c'est pourquoi certaines zones indiquées peuvent être chevauchantes.

### EXEMPLE 2 = Mutations sur gp41 de VIH 1.

On prépare selon les méthodes connues des glycoprotéines d'enveloppe gp41 mutées. Ces mutations sont décrites dans le Tableau 5 annexé qui représente la séquence concernée de gp41 et, alignées sous cette dernière, les séquences mutées. Le niveau des mutations est signalé en soulignant les acides aminés concernés.

On prépare des compositions vaccinales comprenant chacune, en solution saline aqueuse, stérile et apyrogène, l'une des protéines gp41 mutées obtenues ci-dessus.

On immunise des lapins ou des souris avec les protéines mutées obtenues, et on recherche si les anticorps développés par ces animaux reconnaissent ou ne reconnaissent pas l'interleukine-2 humaine, par exemple par la technique ELISA ou PEPSCAN. On sélectionne les protéines mutées qui induisent la formation d'anticorps ne reconnaissant pas l'IL-2, mais reconnaissant la protéine gp41.

La technique PEPSCAN est décrite par J. WORTHINGTON et K. MORGAN, "Epitope mapping using synthetic peptides", in "PEPTIDE ANTIGENS-A practical approach" (G.B. WISDOW Ed.), Oxford University Press (1994).

**TABLEAU 1**

| **LENTIVIRUS** | **HOTE** | **PRINCIPALE CELLULE CIBLE** | |
|---|---|---|---|
| VAIE | Cheval | Macrophage | Anémie hémolytique |
| VISNA VIRUS | Mouton | Macrophage | Visna maedi : encéphalite-pneumonie interstitielle |
| VECA | Chèvre | Macrophage | Immunodéficience - encéphalopathie - arthrite |
| VIB | Bovin | Lymphocyte T | Immunodéficience - lymphocytose bovine |
| VIF | Chat+félidés | Lymphocyte T | Immunodéficience (SIDA) |
| VIS | Primates (singes) | Lymphocyte T | Immunodéficience (SIDA) |
| VIH | Homme | Lymphocyte T | Immunodéficience (SIDA) |

| | | | |
|---|---|---|---|
| VAIE - désigne le virus de l'anémie infectieuse équine VECA désigne le virus de l'encéphalite caprine VIF signifie : virus de l'immunodéficience féline VIS signifie : virus de l'immunodéficience simienne VIH signifie : Virus de l'immunodéficience humaine. | | | |

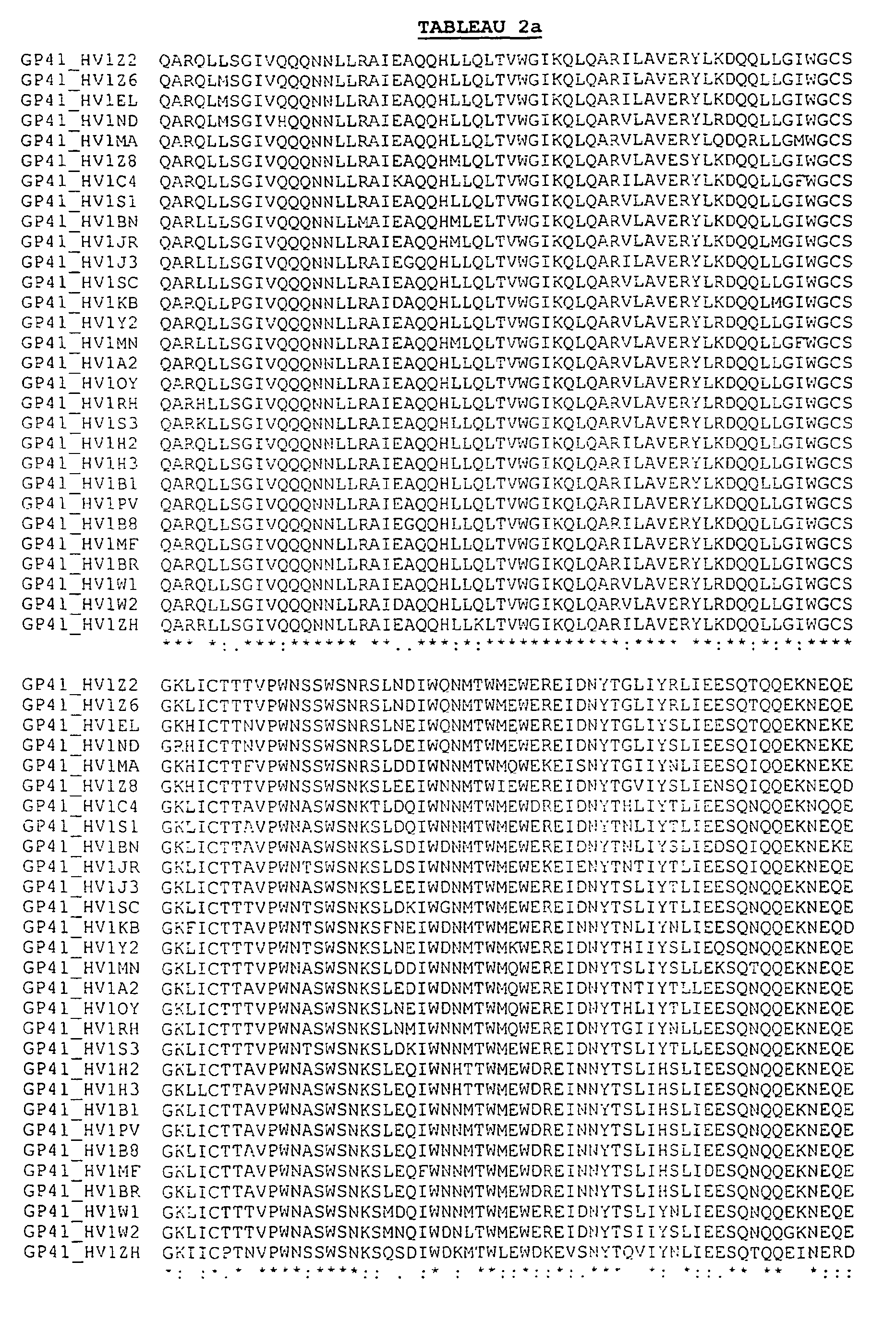

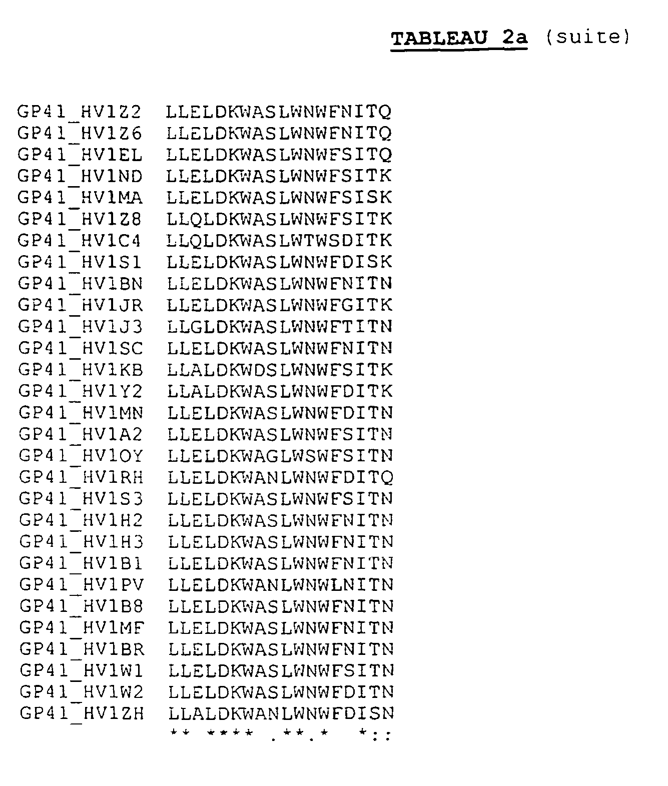

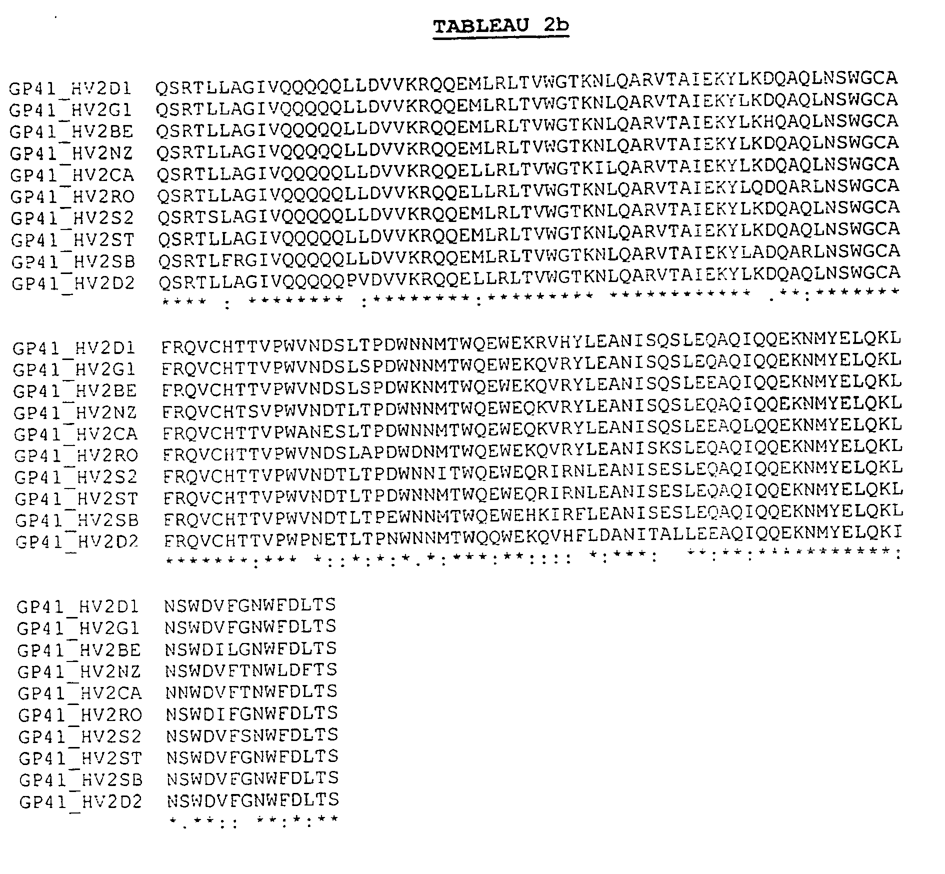

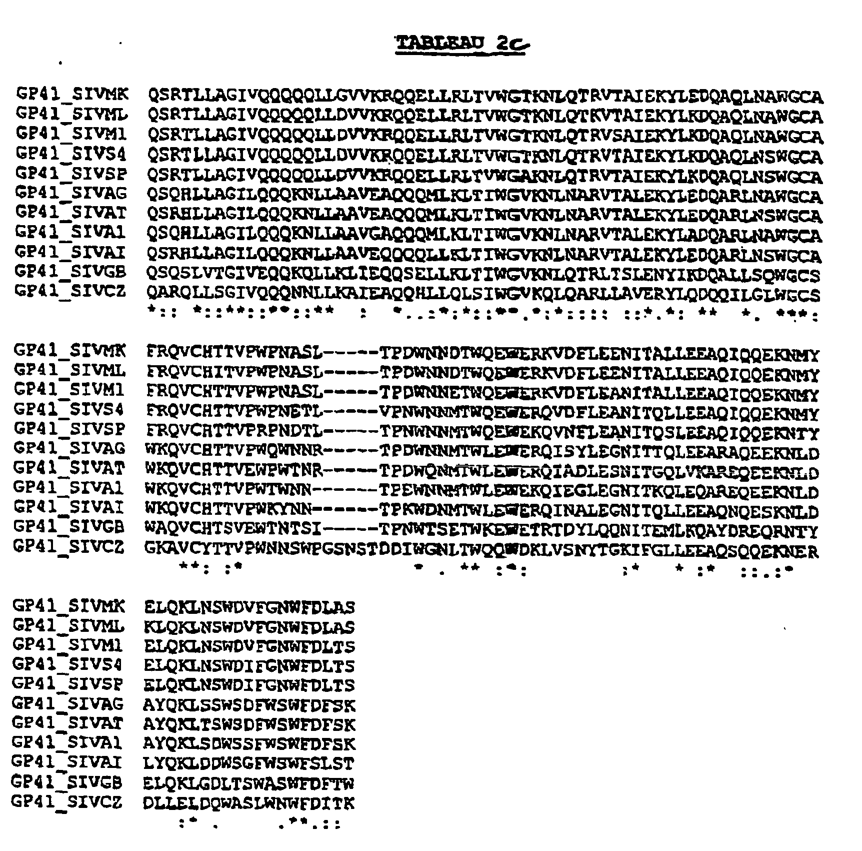

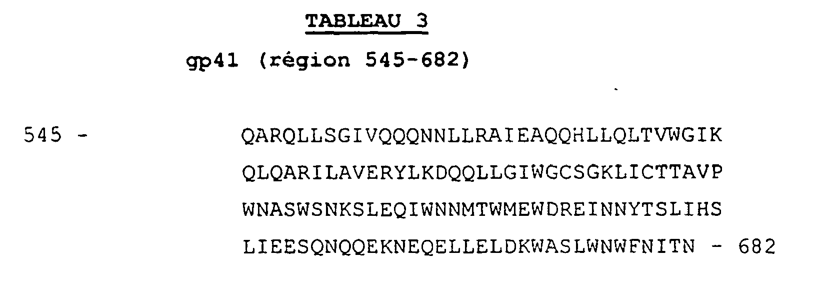

**TABLEAU 3bis**

| **Zones de gp41** | |
|---|---|
| Zone 555-577 | QQQNNLLRAIEAQQHLLQLTVWG |
| Zone 572-601 | QLTVWGIKQLQARILAVERYLKDQQLLGIW |
| Zone 590-620 | RYLKDQQLLGIWGCSGKLICTTAVPWNASWS |
| Zone 628-663 | WNNMTWMEWDREINNYTSLIHSLIEESQNQQEKNEQ |

**TABLEAU 4**

| **IL-2** |
|---|
| Zone 27-47 |
| TKKTQLQLEHLLLDLQMILNG |
| Zone 45-69 |
| LNGINNYKNPKLTRMLTFKFYMPKK |
| Zone 99-121 |
| HLRPRDLISNINVIVLELKGSET |
| Zone 131-153 |
| TATIVEFLNRWITFCQSIISTLT |

**TABLEAU 4Bis**

| **gp41** | **IL-2** |
|---|---|
| Zone 572-601 | Zone 27-47 |
| QLTVWGIKQLQARI**L**AV**E**RY**L**K**D**Q**Q**LLGIW | TKKTQ**L**QL**E**HL**L**L**D**L**Q**MILNG |

**TABLEAU 5**

| **Mutations au niveau de la zone 555-577** | |
|---|---|
| Zone 555-577 | QQQNNLLRAIEAQQHLLQLTVWG |
| Mutation 1: | QQQNNLL**A**AIEAQQHLLQLTVWG |
| Mutation 2: | QQQNNLLRAIE**R**QQHLLQLTVWG |
| Mutation 3: | QQQNNLL**A**AIE**R**QQHLLQLTVWG |
| Mutation 4: | QQQNNLLRAIEAQQ**E**LLQLTVWG |
| Mutation 5: | QQQNNLLRAIEAQQ**Q**LLQLTVWG |
| Mutation 6: | QQQNNLLRAIEAQQHLL**R**LTVWG |
| Mutation 7: | QQQNNLLRAIEAQQHLL**K**LTVWG |
| Mutation 8: | QQQNNLLRAIEAQQ**Q**LL**K**LTVWG |

| **Mutations au niveau de la zone 572-601** | |
|---|---|
| Zone 572-601 | QLTVWGIKQLQARILAVERYLKDQQLLGIW |
| Mutation 1 : | QLTVWGIKQLQARILAVERYLK**A**QQLLGIW |
| Mutation 2 : | QLTVWGIKQLQARILAVE**A**YLK**D**QQLLGIW |
| Mutation 3 : | QLTVTWGIKQLQARILAVE**A**YLK**A**QQLLGIW |
| Mutation 4 : | QLTVWGIKQLQARILAVE**D**YLK**R**QQLLGIW |
| Mutation 5 : | QLTVWGIKQLQARI**T**AVERYLKDQQLLGIW |

| **Mutations au niveau de la zone 590-620** | |
|---|---|
| Zone 590-620 | RYLKDQQLLGIWGCSGKLICTTAVPWNASWS |
| Mutation 1: | **K**YLKDQQLLGIWGCSGKLICTTAVPWNASWS |
| Mutation 2 : | RYLKDQ**A**LLGIWGCSGKLICTTAVPWNASWS |
| Mutation 3 : | RYLKDQQ**Q**LGIWGCSGKLICTTAVPWNASWS |
| Mutation 4 : | RYLKDQ**AQ**LGIWGCSGKLICTTAVPWNASWS |
| Mutation 5: | RYLKDQ**AR**LGIWGCSGKLICTTAVPWNASWS |
| Mutation 6: | RYLKDQQLL**NS**WGCSGKLICTTAVPWNASWS |
| Mutation 7 : | RYLKDQQLLGIWGCS**Q**KLICTTAVPWNASWS |
| Mutation 8 : | RYLKDQQLLGIWGCS**F**KLICTTAVPWNASWS |
| Mutation 9 : | RYLKDQQLLGIWGCSGKLICTTAVPWNAS**S**S |
| Mutation 10 : | RYLKDQQLLGIWGCSGKLICTTAVPWNA**DTL** |
| Mutation 11 : | RYLKDQQLLGIWGCSGKLICTTAVPWNA**TNR** |
| Mutation 12 : | RYLKDQQLLGIWGCSGKLICTTAVPGINA**NTR** |
| Mutation 13: | RYLKDQQLLGIWGCSGKLICTTAVPWNA**NT**S |

| **Mutations au niveau de la zone 628-663** | |
|---|---|
| Zone 628-663 | WNNMTWMEWDREINNYTSLIHSLIEESQNQQEKNEQ |
| Mutation 1 : | WNNMTWMEWDREINNY**E**SLIHSLIEESQNQQEKNEQ |
| Mutation 2 : | WNNMTWMEWDREINNYTS**N**IHSLIEESQNQQEKNEQ |

## Revendications

1. Procédé de recherche et d'obtention d'un vaccin contre les effets pathogènes associés à l'infection d'un hôte, animal ou humain, par un rétrovirus VIH capable de pénétrer dans une cellule cible dudit hôte, ladite cellule cible possédant un récepteur membranaire pour la protéine de l'hôte interleukine-2 (IL-2), dans lequel :
a) on prépare des agents vaccinaux à base d'un polypeptide comprenant au moins une partie de la protéine d'enveloppe gp41 d'une souche pathogène dudit rétrovirus, ledit polypeptide étant présent, dans lesdits agents vaccinaux, sous une forme modifiée,
- ladite partie de la protéine d'enveloppe gp41 étant choisie parmi celles qui comprennent au moins un fragment d'une zone immunodominante de ladite protéine d'enveloppe gp41, ledit fragment contenant au moins un acide aminé qui est un acide aminé conservé de ladite zone immunodominante et qui est présent dans ladite souche pathogène,
- ledit polypeptide étant choisi parmi ceux qui, à l'état non modifié, induisent une réponse immunitaire dirigée à la fois contre ladite zone immunodominante et contre la protéine de l'hôte IL-2, et
b) on sélectionne comme vaccin un tel polypeptide modifié choisi parmi ceux qui induisent une réponse immunitaire dirigée contre ladite zone immunodominante de la protéine d'enveloppe gp41 et non contre la protéine de l'hôte IL-2.

2. Procédé selon la revendication 1, dans lequel ladite zone immunodominante est choisie
- parmi celles qui donnent une réaction croisée, de type B et/ou de type T, avec ladite protéine de l'hôte IL-2, et/ou
- parmi celles pour lesquelles on a déterminé préalablement une analogie de structure tridimensionnelle avec une partie de ladite protéine de l'hôte IL-2.

3. Procédé selon l'une quelconque des revendications précédentes, dans lequel :
- ledit agent vaccinal contient, sous forme modifiée, un polypeptide comprenant au moins une partie de la zone 545-682 de la protéine gp41 de HIV 1.

4. Procédé selon l'une quelconque des revendications précédentes, dans lequel ledit agent vaccinal est un oligomère d'au moins une partie de la protéine d'enveloppe gp41 dudit rétrovirus VIH sous forme modifiée.

5. Procédé selon l'une quelconque des revendications précédentes, dans lequel lesdits polypeptides modifiés sont obtenus par la réalisation de mimotopes.

6. Agent vaccinal pouvant être obtenu selon le procédé de l'une quelconque des revendications 1 à 5.

7. Agent vaccinal contre les effets pathogènes associés à l'infection d'un hôte, animal ou humain, par un rétrovirus VIH,
ledit rétrovirus étant capable de pénétrer une cellule cible dudit hôte possédant un récepteur membranaire pour une protéine de l'hôte interleukine-2 (IL-2),
ledit rétrovirus possédant une protéine d'enveloppe gp41 induisant une réponse immunitaire dirigée à la fois contre une zone conservée et immunodominante de ladite protéine d'enveloppe gp41 et contre ladite protéine de l'hôte IL-2,
ledit agent vaccinal étant capable d'induire une réponse immunitaire dirigée contre ladite zone de ladite protéine d'enveloppe gp41 et non contre ladite protéine IL-2.

8. Agent vaccinal selon la revendication précédente, qui est un mimotope d'au moins une partie de ladite zone conservée et immunodominante.

9. Anticorps pouvant être obtenus par immunisation à l'aide d'un vaccin selon l'une quelconque des revendications 6 à 8, lesdits anticorps reconnaissant ladite protéine d'enveloppe gp41 et non la protéine de l'hôte interleukine-2.

10. Composition pharmaceutique contenant un anticorps selon la revendication 9.

11. Utilisation d'un polypeptide modifié pouvant être obtenu par le procédé de l'une quelconque des revendications 1 à 5, dans la préparation d'une composition vaccinale destinée à prévenir les effets pathogènes associés à l'infection d'un hôte, animal ou humain, par un rétrovirus VIH capable de pénétrer dans une cellule cible dudit hôte, ladite cellule cible possédant un récepteur membranaire pour une protéine de l'hôte interleukine-2, et ledit rétrovirus possédant une protéine d'enveloppe gp41 induisant une réponse immunitaire dirigée à la fois contre une zone conservée et immunodominante de ladite protéine d'enveloppe gp41 et contre ladite protéine de l'hôte interleukine-2.

12. Utilisation d'un agent vaccinal tel que défini dans l'une quelconque des revendications, 6 à 8 dans la préparation d'une composition vaccinale contre ledit rétrovirus.

13. Polynucléotide codant pour un polypeptide modifié tel que défini dans l'une quelconque des revendications 1 à 5, et vecteur d'expression contenant ledit polynucléotide.

## Claims

1. Method of searching for and obtaining a vaccine against the pathogenic effects related to the infection of an animal or human host by a retrovirus HIV capable of penetrating into a target cell of said host, said target cell possessing a membrane receptor for a protein of said host interleukin-2 (IL-2), in which:
a) vaccine agents based on a polypeptide comprising at least part of an envelope protein gp41 of a pathogenic strain of said retrovirus are prepared, said polypeptide being present, in said vaccine agents, in a modified form,
- said part of the envelope protein gp41 being chosen from those which comprise at least one fragment of an immunodominant region of said envelope protein gp41, said fragment containing at least one amino acid which is a conserved amino acid of said immunodominant region and which is present in said pathogenic strain,
- said polypeptide being chosen from those which, in the unmodified state, induce an immune response directed both against said immunodominant region and against the protein of the host IL-2, and
b) there is selected as vaccine such a modified polypeptide chosen from those which induce an immune response directed against said immunodominant region of the envelope protein gp41 and not against the protein of the host IL-2.

2. Method according to claim 1, in which said immunodominant region is chosen
- from those which give a cross-reaction, of the B type and/or of the T type, with said protein of the host IL-2, and/or
- from those for which a three-dimensional structural analogy with part of said protein of the host IL-2 has been determined beforehand.

3. Method according to any one of the preceding claims, in which said vaccine agent contains, in a modified form, a polypeptide comprising at least a part of region 545-682 of the gp41 protein of HIV1.

4. Method according to any one of the preceding claims, in which said vaccine agent is an oligomer of at least part of a transmembrane glycoprotein gp41 of said retrovirus HIV in modified form.

5. Method according to any one of the preceding claims, in which said modified polypeptides are obtained by the production of mimotopes.

6. Vaccine agent which may be obtained according to the method of any one of claims 1 to 5.

7. vaccine agent against the pathogenic effects related to the infection of an animal or human host by a retrovirus HIV,
said retrovirus being capable of penetrating a target cell of said host possessing a membrane receptor for a protein of said host interleukin-2 (IL-2),
said retrovirus possessing an envelope protein gp41 inducing an immune response directed both against a conserved and immunodominant region of said envelope protein gp41 and against said protein of the host IL-2,
said vaccine agent being capable of inducing an immune response directed against said region of said envelope protein gp41 and not against said protein IL-2.

8. Vaccine agent according to the preceding claim, in which the vaccine agent is a mimotope of at least part of said conserved and immunodominant region.

9. Antibody which can be obtained by immunization with the aid of a vaccine according to any one of claims 6 to 8, said antibodies recognizing said envelope protein gp41 and not the protein of the host interleukin-2 (IL-2).

10. Pharmaceutical composition containing an antibody according to claim 9.

11. Use of a modified polypeptide which can be obtained by the method according to any one of claims 1 to 5, in the preparation of a vaccine composition for preventing the pathogenic effects related to the infection of an animal or human host by a retrovirus VIH capable of penetrating into a target cell of said host, said target cell possessing a membrane receptor for a protein of the host interleukin-2 (IL-2), and said retrovirus possessing an envelope protein gp41 inducing an immune response directed both against a conserved and immunodominant region of said envelope protein gp41 and against said protein of the host IL-2.

12. Use of a vaccine agent as defined in any one of claims 6 to 8 in the preparation of a vaccine composition against said retrovirus.

13. Polynucleotide encoding a modified polypeptide as defined in any one of claims 1 to 5, and expression vector containing said polynucleotide.

## Patentansprüche

1. Verfahren zur Suche und Gewinnung eines Impfstoffs gegen die pathogenen Wirkungen, die mit der Infektion eines tierischen oder menschlichen Wirts durch ein HIV-Retrovirus, das dazu in der Lage ist, in eine Target-Zelle des Wirts einzudringen, verbunden sind, welche Target-Zelle einen Membran-Rezeptor für das Wirts-Interleukin-2 (IL-2)-Protein aufweist, gemäß dem man:
a) impfende Mittel bildet auf der Grundlage eines Polypeptids, das mindestens einen Teil des Hüll-Proteins gp41 eines pathogenen Stamms des Retrovirus umfaßt, welches Polypeptid in einer modifizierten Form in den impfenden Mitteln vorliegt,
- wobei der Teil des Hüll-Proteins gp41 aus jenen ausgewählt wird, die mindestens ein Fragment eines immunodominierenden Bereichs des Hüll-Proteins gp41 umfassen, welches Fragment mindestens eine Aminosäure enthält, die eine Aminosäure aus dem immunodominierenden Bereich ist und die in dem pathogenen Stamm vorhanden ist,
- welches Polypeptid aus jenen ausgewählt ist, die in nicht-modifiziertem Zustand eine Immunantwort induzieren, die gleichzeitig gegen den immunodominierenden Bereich und gegen das Wirts-IL-2-Protein gerichtet ist, und
b) als Impfstoff ein solches modifiziertes Polypeptid aus jenen auswählt, die eine Immunantwort induzieren, welche gegen den immunodominierenden Bereich, jedoch nicht gegen das Wirts-IL-2-Protein gerichtet ist.

2. Verfahren nach Anspruch 1, worin der immunodominierende Bereich ausgewählt ist
- aus jenen, welche eine Kreuzreaktion des Typs B und/oder des Typs T mit dem Wirts-IL-2-Protein ergeben, und/oder
- aus jenen, für die man zuvor eine dreidimensionale Strukturanalogie mit einem Teil des Wirts-IL-2-Proteins festgestellt hat.

3. Verfahren nach einem der vorhergehenden Ansprüche, worin:
- das impfende Mittel in modifizierter Form ein Polypeptid enthält, welches mindestens einen Teil des Bereichs 545-682 des Proteins gp41 von HIV 1 enthält.

4. Verfahren nach einem der vorhergehenden Ansprüche, worin das impfende Mittel ein Oligomer mindestens eines Teils des Hüllproteins gp41 des HIV-Retrovirus in modifizierter Form ist.

5. Verfahren nach einem der vorhergehenden Ansprüche, worin die modifizierten Polypeptide durch die Bildung von Mimotopen erhalten worden sind.

6. Impfendes Mittel, erhältlich nach dem Verfahren gemäß einem der Ansprüche 1 bis 5.

7. Impfendes Mittel gegen die pathogenen Wirkungen, die mit der Infektion eines tierischen oder menschlichen Wirts durch ein HIV-Retrovirus verbunden sind,
welches Retrovirus dazu in der Lage ist, in eine Target-Zelle des Wirts einzudringen, welche Target-Zelle einen Membran-Rezeptor für das Wirts-Interleukin-2 (IL-2) aufweist,
welches Retrovirus ein Hüllprotein gp41 besitzt, welches eine Immunantwort induziert, die gleichzeitig gegen einen beibehaltenden und immunodominierenden Bereich des Hüllproteins gp41 und gegen das Wirts-IL-2-Protein gerichtet ist,
welches impfende Mittel dazu in der Lager ist, eine Immunantwort zu induzieren, die gegen den Bereich des Hüllproteins gp41 und nicht gegen das Protein IL-2 gerichtet ist.

8. Impfendes Mittel nach dem vorhergehenden Anspruch, welches ein Mimotop mindestens eines Teils des beibehaltenden und immunodominierenden Bereichs ist.

9. Antikörper, erhältlich durch Immunisierung mit Hilfe eines Impfstoffs nach einem der Ansprüche 6 bis 8, welche Antikörper das Hüllprotein gp41 und nicht das Wirts-Interleukin-2-Protein erkennen.

10. Pharmazeutische Zubereitung, enthaltend einen Antikörper nach Anspruch 9.

11. Verwendung eines modifizierten Polypeptids, welches nach dem Verfahren nach einem der Ansprüche 1 bis 5 erhältlich ist, für die Herstellung einer Impfzubereitung zur Vorbeugung der pathogenen Wirkungen, die mit der Infektion eines tierischen oder menschlichen Wirts durch ein HIV-Retrovirus, das dazu in der Lage ist, in eine Target-Zelle des Wirts einzudringen, verbunden sind, welche Target-Zelle einen Membran-Rezeptor für das Wirts-Interleukin-2-Protein aufweist, und welches Retrovirus ein Hüllprotein gp41 aufweist, welches eine Immunantwort induziert, die gleichzeitig gegen einen beibehaltenden und immunodominierenden Bereich des Hüllproteins gp41 und gegen das Wirts-Interleukin-2-Protein gerichtet ist.

12. Verwendung eines impfenden Mittels nach einem der Ansprüche 6 bis 8 bei der Herstellung einer impfenden Zubereitung gegen das Retrovirus.

13. Polynucleotid, welches für ein modifiziertes Polypeptid, wie es in einem der Ansprüche 1 bis 5 definiert ist, codiert, und Expressionsvektor enthaltend das Polynucleotid.
